(19) Europäisches Patentamt
European Patent Office
Office européen des brevets

(11) Publication number: **0 328 834**
**A1**

(12) # EUROPEAN PATENT APPLICATION

(21) Application number: **88400348.4**

(22) Date of filing: **16.02.88**

(51) Int. Cl.4: **C07F 9/38 , C07F 9/40**

(43) Date of publication of application:
**23.08.89 Bulletin 89/34**

(84) Designated Contracting States:
**FR**

(71) Applicant: **MERRELL DOW PHARMACEUTICALS INC.**
**2110 East Galbraith Road**
**Cincinnati Ohio 45215-6300(US)**

(72) Inventor: **Halazy, Serge**
**2, rue Hans Haug Wolfisheim**
**F-67200 Strasbourg(FR)**
Inventor: **Danzin, Charles**
**18, rue Geiler**
**F-67000 Strasbourg(FR)**

(74) Representative: **Gillard, Marie-Louise et al**
**Cabinet Beau de Loménie 55, Rue d'Amsterdam**
**F-75008 Paris(FR)**

(54) **Novel aspartate transcarbamylase inhibitors.**

(57) This invention relates to halogenated derivatives of certain phosphonates having the ability to inhibit aspartate transcarbamylase and therefore are useful in the treatment of certain cancers.

EP 0 328 834 A1

## NOVEL ASPARTATE TRANSCARBAMYLASE INHIBITORS

This invention relates to halogenated derivatives of certain phosphonates having the ability to inhibit aspartate transcarbamylase and therefore are useful in the treatment of certain cancers.

More specifically this invention relates to novel halogenated derivatives of phosphonates having the structural formula

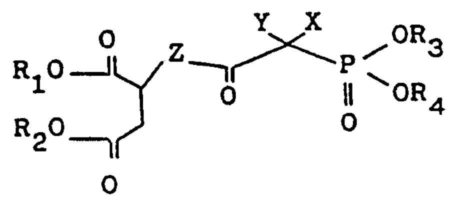

I

or a pharmaceutically acceptable salt thereof, wherein

Z is NH or $CH_2$

X,Y is F,F, H,F, Cl,Cl, Cl,H,

$R_1$ and $R_2$ each are H or $C_{1-6}$ alkyl,

$R_3$ and $R_4$ each are H or $C_{1-6}$ lower alkyl.

It is to be noted that the compounds may exist in their enantiomeric forms and thus the individual enantiomers as well as the mixtures thereof are contemplated as part of the scope of this invention.

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intended to apply to any non-toxic organic or inorganic acid addition salts of the base compounds of formula I. Illustrative inorganic acids which form suitable salts include hydrochloric, hydrobromic, sulfuric and phosphoric acids and acid metal salts such as sodium monohydrogen orthophosphate and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic and 2-phenoxybenzoic acids. Other organic acids which form suitable salts are the sulfonic acids such as methane sulfonic acid and 2-hydroxyethane sulfonic acid. Either the mono- or the diacid salts can be formed, and such salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic organic solvents and which, in comparison to their free base forms, demonstrate higher melting points and an increased solubility.

In general the compounds of formula I may be prepared by methods and techniques which are analogously known in the art. Of course the specific route followed for the preparation of any one specific compound will depend upon many factors (e.g. availability and costs of starting materials, protecting groups and such other factors generally appreciated by the skilled artisan) but, in general, two generic routes of synthesis are available depending primarily upon the definition of the moiety defined by Z.

In those instances wherein Z is NH it is convenient to utilize derivatives of phosphono acetic acid and aspartic acid according to the following reaction scheme

Reaction Scheme A

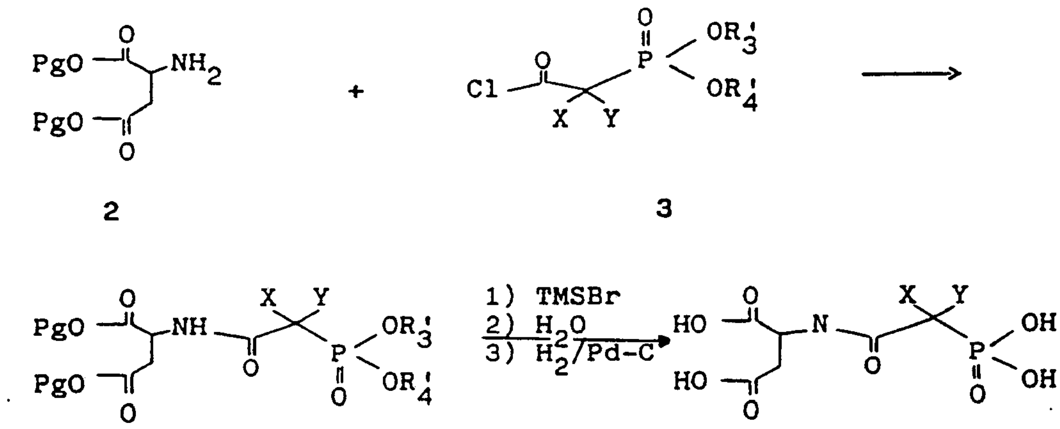


2 3

4 5

wherein $R'_3$ and $R'_4$ are $C_{1-6}$ alkyl, and Pg is a suitable protecting group, preferably benzyl, methyl or ethyl.

The initial condensation reaction of the acid chloride (3) with suitably protected derivatives of aspartic acid (2) is readily achieved by contacting equivalent quantities of the reactants together at temperatures of about -30° C to 30° C, preferably at about 0° C in the presence of a base such as dimethylaminopyridine (DMAP), N-methyl morpholine (NMM), triethylamine (TEA) and the like; the reaction taking place under anhydrous conditions in a polar aprotic solvent, preferably dichloromethane. Of course, the acid chloride derivatives of formula 3 are readily prepared from the appropriate $OR'_3$ , $OR'_4$ phosphono-X,Y-substituted acetic acid derivatives by standard procedures well known in the art. In general, these acids are reacted with oxalyl chloride in the presence of a base (e.g. DMAP, NMM, TEA and the like) at temperatures of about -10° C to 20° C, preferably at 0° C under an inert atmosphere (argon or nitrogen being preferred) in a polar aprotic solvent under anhydrous conditions according to well known conditions.

Following the above-described condensation the $R'_3$ , $R'_4$ protecting groups (preferably methyl and ethyl) are selectively removed by reaction with trimethylsilyl bromide (TMSBr) in a polar aprotic solvent (preferably $CH_2Cl_2$) and the resulting products are de-protected (e.g. de-benzylated) by hydrogenolysis, preferably by treatment with hydrogen in the presence of a catalyst, such as palladium on carbon in water. In the event the protecting groups (represented by Pg) are lower alkyl, then they are cleaved by basic hydrolysis.

The preparation of the generic class of compounds of formula I wherein Z represents a $CH_2$ moiety is achieved by a nucleophilic addition of an appropriate aldehyde with a lithio derivative of a diester of an X,Y-substituted methyl phosphonate to form an alcohol which is oxidized to its corresponding ketone. Following this oxidation the hydroxyl-protecting groups are removed as described above. This sequence is depicted by the following reaction scheme

Reaction Scheme B

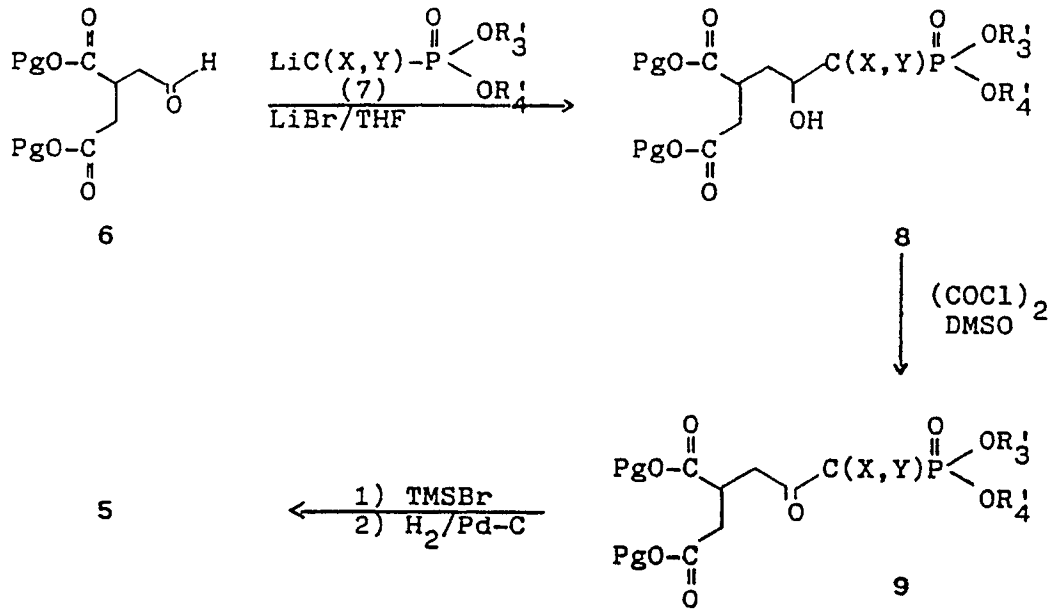


6 8 9 5

wherein Pg, X, Y, $R_3'$ and $R_4'$ are as previously described.

The nucleophilic addition of the aldehydes (6) is effected with 2 equivalents of the $R_3'$, $R_4'$, X, Y-substituted lithio methanephosphonates at low temperatures of about -50°C to -110°C, preferably about -78°C in a THF/ether (3/1) solvent mixture in the presence of 1 equivalent of lithium bromide.

The reaction is preferably effected under an inert atmosphere, preferably using nitrogen or argon, under anhydrous conditions in an aprotic polar solvent. Following completion the reaction is generally quenched with aqueous ammonium chloride and the resulting product isolated according to standard procedures. The so-obtained alcohols (8) are subjected to a Swern oxidation procedure to the corresponding ketones. The oxidation is conducted in an aprotic solvent under an inert atmosphere (nitrogen or argon preferred) under anhydrous conditions using dimethylsulfoxide (DMSO) in the presence of an agent activating DMSO as for example oxalylchloride or trifluoroacetic anhydride, followed by the addition of a base, preferably TEA. Following this oxidation the hydroxyl-protecting groups of the compounds of formula (9) are removed by sequential treatment with trimethylsilyl bromide and hydrogenolysis by the procedures described for Reaction Scheme A. In those instance wherein Pg is methyl or ethyl, then those protecting groups are removed by basic hydrolysis.

The necessary aldehydes of formula 6 are readily prepared by subjecting 3-cyclopentene-1-carboxylate esters (benzyl, methyl or ethyl) to ozonolysis using gaseous ozone according to the procedure outlined in Organic Synthesis, Vol. 64, A.S. Kende (editor), pp. 150-156 in an article submitted by R.E. Claus and S.L. Schreiber. In this procedure the ozonolytic cleavage is effected in the presence of an alcohol to produce a compound having an aldehyde and a methoxy hydroperoxide group at the termini. (Of course, if benzyl alcohol is used a benzyloxy hydroperoxide is formed instead of the methoxy hydroxyperoxide. Thus, it is preferred to use that alcohol which will best accomodate its removal after the Swern oxidation). Following the ozonolysis, in situ-dehydration, preferably with acetic anhydride in the presence of base (preferably TEA), affords the desired starting materials (6).

It is preferred to produce compounds of formula I in their L-enantiomeric form and by the use of L-aspartic acid derivatives (such as in the Reaction Scheme A) such compounds are readily prepared. Those compounds produced by Reaction Scheme B will be produced as racemic mixtures which can readily be separated by procedures well known in the art. In general it is preferred to separate the enantiomeric forms of the aldehydes of formula 6 rather than after they have been converted to compounds (5).

In practice it is preferred to utilize phosphono esters wherein $R_3'$ and $R_4'$ are methyl or ethyl. Reactants utilizing other alkyl esters may be utilized and such esters may be prepared by methods analogous to those used in the preparation of intermediates (3) and (7).

The following examples are illustrative of the above-described procedures for the preparation of the

compounds of formula I.

## Example I

Preparation of

### 2-[(2,2-DIFLUORO-1-OXO-2-PHOSPHONO ETHYL)AMINO]BUTANEDIOIC ACID

#### Step A:

2-[(2-DIETHOXYPHOSPHINYL-2,2-DIFLUORO-1-OXO-ETHYL)AMINO]BUTANEDIOIC ACID, BIS-(PHENYLMETHYL) ESTER

1.75 ml of oxalyl chloride (20 mmol) are slowly added to a stirred solution of diethyl phosphono-difluoro-acetic acid (20 mmol, 4.64 g) and 2.02 g of N-methylmorpholine (20 mmol)) dissolved in 50 ml of anhydrous dichloromethane at 0 °C under argon. The reaction mixture is cooled to 0 °C and 9.7 g of aspartic acid tosylate (20 mmol) and 4.5 g of N-methylmorpholine (43 mmol) dissolved in 100 ml of anhydrous dichloromethane are added. After stirring for 18 hours at 20 °C the reaction mixture is hydrolyzed with saturated aqueous bicarbonate (30 ml), washed with 1 N HCl (30 ml), bicarbonate (30 ml) and brine (30 ml). The organic layer is dried over $Na_2SO_4$, filtered and purified by flash chromatography on silica gel giving 4.8 g of 2-[(2-diethoxyphosphinyl-2,2-difluoro-1-oxo-ethyl)amino]butanedioic acid bis-(phenylmethyl) ester.

#### Step B:

2-[(2,2-DIFLUORO-1-OXO-2-PHOSPHONO ETHYL)AMINOJBUTANEDIOIC ACID, BIS(PHENYLMETHYL) ESTER

10 ml of trimethylsilylbromide (75 mmol) are slowly added to a stirred solution of 2-[(2-diethoxyphosphinyl-2,2-difluoro-1-oxo-ethyl)amino]butanedioic acid bis(phenylmethyl) ester (4 g, 7.5 mmol) dissolved in 50 ml of anhydrous dichloromethane at 0 °C under argon. The reaction mixture is stirred at 20 °C for 18 hours and evaporated to dryness. The residue is dissolved in 25 ml of acetonitrile and hydrolyzed by 1.5 ml of water. The reaction mixture is evaporated to dryness to give 3.4 g of 2-[(2,2-difluoro-1-oxo-2-phosphono ethyl)amino]butanedioic acid, bis(phenylmethyl) ester whioh will be used in the next step without further purification.

#### Step C:

2-[(2,2-DIFLUORO-1-OXO-2-PHOSPHONO ETHYL)AMINO]BUTANEDIOIC ACID

3.4 g of 2-[(2,2-difluoro-1-oxo-2-phosphono ethyl)amino]butanedioic acid, bis(phenylmethyl) ester (7.2 mmol) are dissolved in water (70 ml) and stirred under atmospheric pressure of hydrogen in the presence of 0.6 g of Palladium dispersed on charcoal. After 18 hours, the reaction mixture is filtered and 2-[(2.2-difluoro-1-oxo-2-phosphono ethyl)amino]butanedioic acid is obtained on crystallization from THF (tetrahydrofuran).

## Example II

## Preparation of

## (3,3-DICHLORO-2-OXO-3-PHOSPHONO PROPYL)BUTANEDIOIC ACID

### Step A:

### 3,4-BUTANAL DICARBOXYLIC ACID, BIS(PHENYLMETHYL)ESTER

A round-bottomed flask equipped with a glass tube, a calcium chloride drying tube and a glass stopper is charged with 20 g of methyl-B-cyclopentene-1-carboxylate (158 mmol), 500 ml of dichloromethane, 100 ml of methanol and 4 g of anhydrous sodium bicarbonate. Ozone is bubbled through the solution at -78° C until a blue color appears. Nitrogen is passed through the solution until the blue color is discharged and the mixture is allowed to be stirred at 20° C. The solution is filtered and 150 ml of benzene are added. The volume is reduced to approximately 100 ml by evaporation. After dilution with 450 ml of dichloromethane, the flask is cooled to 0° C and 32 ml of triethylamine and 43 ml of acetic anhydride are added dropwise and the solution is stirred under a nitrogen atmosphere for 30 min. The ice-bath is removed and stirring is continued for 4 hours. The solution is washed with 150 mlportions of aqueous 0.1 N HCl, aqueous 10% NaOH and water. The organic layer is dried ($Na_2SO_4$), filtered and evaporated to give 25 g of crude material which is purified by flash chromatography on silica gel affording 18 g of 3,4-butanal dicarboxylic acid, bis(phenylmethyl) ester.

### Step B:

### 2-(3-DIETHOXYPHOSPHINYL-3,3-DICHLORO-2-HYDROXYPROPYL)BUTANEDIOIC ACID, DIMETHYL ESTER

A solution of diethyl trichloromethane phosphonate (25.6 g) together with 5 g of lithium bromide in ether (90 ml) and THF (70 ml) is placed in a four-necked flask equipped with a stirrer, an addition funnel, a low-T° thermometer and a nitrogen inlet tube. A 1.5 N n-butyllithium solution (72 ml) is added over a 10 min period, dropwise at -100° C under $N_2$ atmosphere. After the addition is complete the temperature of the mixture is allowed to rise to -85° C for 10 min. Then a solution of 10 g of 3,4-butanal dicarboxylic acid, bis-(phenylmethyl) ester in 30 ml of THF and 20 ml of ether is slowly added to the reaction mixture kept between -95° C to -110° C. The mixture is stirred at -100° C for 30 min, hydrolyzed with rapid addition of 1 N $H_2SO_4$, warmed up and extracted with ethylacetate (3 times 100 ml). The extract is washed with 0.1 N $H_2SO_4$, brine, dried over sodium sulfate, filtered and evaporated to give a residue which is purified by flash chromatography on silica gel giving 11 g of 2-(3-diethoxyphosphinyl-3,3-dichloro-2-hydroxypropyl)-butanedioic acid, dimethyl ester.

### Step C:

### 2-(3,3-DICHLORO-3-DIETHOXYPHOSPHINYL-2-OXO-PROPYL)BUTANEDIOIC ACID, DIMETHYL ESTER

DMSO (3.4 ml) is added slowly to a stirred solution of oxalylchloride (2.1 ml) in 20 ml of anhydrous dichloromethane at -78° C under argon. The reaction mixture is stirred at -50° C for 10 min, cooled again to -78° C and a solution of 2-(3-diethoxyphosphinyl-3,3-dichloro-2-hydroxypropyl)butanedioic acid, dimethyl ester (8 g) in 30 ml of anhydrous dichloromethane is added dropwise. After 15 min at -40° C and 1 hour at

20° C, a saturated aqueous ammonium chloride solution is added. The organic layer is separated, the aqueous phase is extracted twice with 80 ml of dichloromethane, the organic phases are combined, washed with brine, dried ($Na_2SO_4$), filtered and evaporated to give 8.7 g of crude material which is purified by flash chromatography on silica gel affording 6.7 g of 2 (3,3-dichloro-3-diethoxyphosphinyl-2-oxo-propyl)-butanedioic acid, dimethyl ester.

Step D:

(3,3-DICHLORO-2-OXO-3-PHOSPHONO PROPYL)BUTANEDIOIC ACID

12 ml of trimethylsilylbromide are slowly added to a stirred solution of 2-(3,3-dichloro-3-diethoxyphosphinyl2-oxo-propyl)butanedioic acid, dimethyl ester (6 g), dissolved in 75 ml of anhydrous dichloromethane at 0° C under argon. The reaction mixture is stirred at 20° C for 18 hours and evaporated to dryness. The residue is dissolved in 30 ml of acetonitrile and hydrolyzed by 2 ml of water. The reaction mixture is evaporated to dryness, affording 4.4 g of product which is dissolved in MeOH (methanol) (30 ml) and water (10 ml) and treated with 0.5 g of LiOH (lithium hydroxide) at 20° C for 15 hours. The reaction mixture is evaporated to dryness. The residue is dissolved in 0.5 N HCl (50 ml), concentrated to a few ml and extracted 5 times by ethylacetate. The organic layers are combined, washed with brine, dried ($Na_2SO_4$), filtered and evaporated to give 4 g of (3,3-dichloro-2-oxo-3-phosphono propyl)butanedioic acid whioh is recrystallized from hot THF.

## Example III

Preparation of

2-(3-DIETHOXYPHOSPHINYL-3,3-DIFLUORO-2-HYDROXY-PROPYL)BUTANEDIOIC ACID, DIMETHYL ESTER

30 mmol of 1.5 N n-butyllithium in hexane (200 ml) are added at -35° C to a stirred solution of 33 mmol of diisopropylamine (33.3 g) in 300 ml of anhydrous THF. The reaction mixture is stirred at 0° C for 30 min, cooled to -78° C and added in 30 min to a stirred solution of 52.5 g of diethyl difluoromethane phosphonate in 300 ml of THF cooled at -78° C under argon. Butanal 3,4-dicarboxylic acid bis methyl ester (34 g) dissolved in 250 ml of THF is then added at -78° C and the reaction mixture is slowly warmed up to 20° C. The brown solution cooled to 0° C is quenched with 0.5 1 of saturated aqueous ammonium chloride and extracted with ethylacetate, washed with 0.5 N HCl, brine, dried ($Na_2SO_4$), filtered and evaporated. The crude product is purified by flash chromatography on silica gel, giving 38 g of 2-(3-diethoxyphosphinyl-3,3-difluoro-2-hydroxypropyl)butanedioic acid, dimethyl ester.

## Example IV

Preparation of

2-(3-DIETHOXYPHOSPHINYL-3-FLUORO-2-HYDROXY-PROPYL)BUTANEDIOIC ACID, BIS METHYL ESTER

200 mmol of 1.5 N n-butyllithium in hexane (130 ml) are added at -35 C to a stirred solution of

diisopropylamine (21 g) in 200 ml of anhydrous THF. The reaction mixture is stirred at 0 °C for 30 min. To this solution cooled at -78 °C is added dropwise diethylfluoromethane phosphonate (34 g) dissolved in 60 ml of anhydrous THF. After stirring for 15 min, butanal 3,4-dicarboxylic acid, bis methyl ester (22 g) dissolved in 50 ml of THF, was added dropwise to the reaction mixture at -78 °C. The reaction mixture was slowly warmed up to 20 °C, quenched with saturated aqueous ammonium chloride, extracted with ethyl acetate (3 x 250 ml), washed with 0.1 N HCl and brine, dried ($Na_2SO_4$), filtered and evaporated. The crude product was purified by flash chromatography on silica gel affording 20 g of 2-(3-diethoxyphosphinyl-3-fluoro-2-hydroxypropyl)butanedioic acid, bis methyl ester.

By following the foregoing procedure there may also be prepared the following compounds:

2-[(2.2-dichloro-1-oxo-2-phosphono ethyl)amino]butanedioic acid
2-(fluoro-1-oxo-2-phosphono ethyl)aminobutanedioic acid
2-(chloro-1 oxo-2-phosphono ethyl)aminobutanedioic acid
(3,3-difluoro-2-oxo-3-phosphono propyl)butanedioic acid
(3-fluoro-2-oxo-3-phosphono propyl)butanedioic acid
(3-chloro-2-oxo-3-phosphono propyl)butanedioic acid

The rational design of effective antineoplastic agents has long been an important adjuvant to classical approaches to medicinal chemistry and biochemical pharmacology. Indeed, this approach has led to the discovery of such clinically active antineoplastic agents as 5-fluorouracil, cyclophosphamide and malphalan. Following such successes, efforts have also been expended to discover antineoplastic agents based on their ability to serve as transition-state inhibitors of aspartate transcarbamylase, an early enzyme in the pathway of de novo pyrimidine nucleotide biosynthesis. This is a particularly desirable approach because some drugs which inhibit this pathway have proven useful in the treatment of a number of human tumor types.

Studies have shown that N-(phosphonoacetyl)-L-aspartate is an effective inhibitor of aspartate transcarbamylase and has been found to be useful in the conjunctive treatment of cancer with such agents as 5-fluorouracil (5-FU), 1-β-D-arabinofuranosylcytosine (ARA-C), methotrexate (MTX) (alone or with 5-FU) and 4′-(9-acridinylamino)methanesulfon)-m-aniside (AMSA) in such cancer disease states as colorectal cancers, malignant melanoma, ovary tumors, neurofibrosarcoma, metastatic adenocarcinoma, and brain tumors such as meningioma and glioblastoma.

N-(Phosphonoacetyl)L-asparate (PALA) is effective in conjunctive therapy in the treatment of the cancer disease states at doses of about 100 mg/m2 to 3000 mg/m2 (e.g., 100 milligrams per square meter of body surface), preferably at 1 to 2 grams/m2 in a single dose scheduled or in 0.5 mg/m2 per day for five days (repeated every 2 weeks) in a multiple dose schedule with the dose schedules of 5-FU, ARA-C, MTX and AMSA. For example, at the aforementioned dose levels for PALA, effective conjunctive therapy in the treatment of the foregoing cancer disease states can be achieved with 5-FU when 5-FU is intravenously (i.v.) administered between 200 and 500 mg/m2 in a single weekly dose, with ARA-C when ARA-C is i.v. administered at 200 to 500 mg/m2, with AMSA when AMSA is i.v. administered at a monthly dose of 90 to 120 mg/m2.

In their use in conjunctive therapy with known anti-neoplastic agents, (e.g., 5-FU, ARA-C, MTX and AMSA) the compounds of formula I may he administered at doses of about 1/5 to 1/1 the doses of PALA when used in conjunctive therapy with the above-mentioned dose regimes for 5-FU, ARA-C, MTX and AMSA.

The amount of the active ingredient to be administered can vary widely according to the particular dosage unit employed, the period of treatment, the age and sex of the patient treated and the nature and extent of the disorder treated. The total amount of the active ingredient to be administered will generally range from about 1 mg/kg to 50 mg/kg and preferably from 3 mg/kg to 25 mg/kg. A unit dosage may contain from 25 to 500 mg of active ingredient, and can be taken one or more times per day. The active compound of formula I can be administered with a pharmaceutical carrier using conventional dosage unit forms either orally, parenterally or topically.

The preferred route of administration is oral administration. For oral administration the compounds can be formulated into solid or liquid preparations such as capsules, pills, tablets, troches, lozenges, melts, powders, solutions, suspensions or emulsions. The solid unid dosage forms can be a capsule which can be of the ordinary hard- or soft-shelled gelatin type containing, for example, surfactants, lubricants and inert fillers such as lactose, sucrose, calcium phosphate and cornstarch. In another embodiment the compounds of this invention can be tableted with conventional tablet bases such as lactose, sucrose and cornstarch in combination with binders such as acacia, cornstarch or gelatin, disintegrating agents intended to assist the break-up and dissolution of the tablet following administration such as potato starch, alginic acid, corn starch and guar gum, lubricants intended to improve the flow of tablet granulations and to prevent the

adhesion of tablet material to the surfaces of the tablet dies and punches, for example, talc, stearic acid or magnesium, calcium or zinc stearate, dyes, coloring agents and flavoring agents intended to enhance the aesthetic qualities of the tablets and make them more acceptable to the patient. Suitable excipients for use in oral liquid dosage forms include diluents such as water and alcohols, for example, ethanol, benzyl alcohol. and the polyethylene alcohols, either with or without the addition of a pharmaceutically acceptably surfactant, suspending agent or emulsifying agent.

The compounds of this invention may also be administered parenterally, that is, intravenously or intramuscularly, as injectable dosages of the compound in a physiologically acceptable diluent with a pharmaceutically carrier which can be a sterile liquid or a mixture of liquids such as water, saline, aqueous dextrose and related sugar solutions, an alcohol such as ethanol, isopropanol or hexadecyl alcohol, glycols such as propylene glycol or polyethylene glycolic glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as soap or a detergent, suspending agent such as pectin, carbomers, methylcellulose, hydroxypropylmethylcellulose or carboxymethylcellulose, or emulsifying agents and other pharmaceutical adjuvants. Illustrative of oils which can be used in the parenteral formulations of this invention are those of petroleum, animal, vegetable or synthetic origin, for example, peanut oil, soybean oil, sesame oil, cottonseed oil, corn oil, olive oil, petrolatum and mineral oil. Suitable fatty acids include oleic acid, stearic acid and isostearic acid. Suitable fatty acid esters are, for example, ethyl oleate and isopropyl myristate. Suitable soaps include fatty alkali metal, ammonium and triethanolamine salts, and suitable detergents include cationic detergents, for example, dimethyl dialkyl ammonium halides, alkyl pyridinium halides, and alkylamines acetates; anionic detergents, for example, alkyl, aryl and olefin sulfonates, alkyl, olefin, ether and monoglyceride sulfates and sulfosuccinates; non-ionic detergents, for example, fatty amine oxides, fatty acid alkanolamides, and polyoxyethylenepolypropylene copolymers; and amphoteric detergents, for example, alkyl-beta-aminopropionates and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by condensation of propylene oxide with propylene glycol.

The active ingredient may also be administered by means of a sustained release system whereby the compound of formula I is gradually released at a controlled, uniform rate to form an inert or bioerodible carrier by means of diffusion, osmosis or disintegration of the carrier during the treatment period. Controlled release drug delivery systems may be in the form of a patch or bandage applied to the skin or to the buccal, sublingual or intranasal membranes, an ocular insert placed in the cul de sac of the eye, or a gradually eroding tablet or capsule or a gastrointestinal reservoir administered orally. Administration by means of such sustained release delivery systems permits the tissues of the body to be exposed constantly for a prolonged time period to a therapeutically or prophylactically effective dosage of a compound of formula I. The unit dosage of the compound administered by means of a sustained release system will approximate the amount of an effective daily dosage multiplied by the maximum number of days during which the carrier is to remain on or in the body of the host. The sustained release carrier may be in the form of a solid or porous matrix or reservoir and may be formed from one or more natural or synthetic polymers, including modified or unmodified cellulose, starch, gelatin, collagen, rubber, polyolefins, polyamides, polyacrylates, polyalcohols, polyethers, polyesters, polyurethanes, polysulphones, polysiloxanes and polyimides as well as mixtures and copolymers of these polymers. The compounds of formula I may be incorporated in the sustained release carrier in a pure form or may be dissolved in any suitable liquid or solid vehicle, including the polymer of which the sustained release carrier is formed.

In the use of the compounds it is preferred to use compounds wherein both X and Y are fluoro or chloro. When one of X or Y is hydrogen it is preferred that the other is fluoro.

European Patent Office

# EUROPEAN SEARCH REPORT

Application Number

EP 88 40 0348

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF THE CHEMICAL SOCIETY, Perkin Transactions I, 1980, pages 2721-2727; J.J. GOODSON et al.: "Inhibitors of pyrimidine biosynthesis, part 1. Synthesis of potential transition-state analogoues of aspartate transcarbamylase" --- | 1 | C 07 F 9/38<br>C 07 F 9/40 |
| A | CHEMICAL ABSTRACTS, volume 103, November 1985, abstract no. 160819x; G.K. FARRINGTON et al.: "Design and synthesis of new transition-state analog inhibitors of aspartate transcarbamylase"; & J. MED. CHEM., 1985, 28 (11), 1668-73 --- | 1 | |
| A | CHEMICAL ABSTRACTS, volume 85, no. 23, 6th December 1976, page 201, abstract no. 173460h; M.F. ROBERTS et al.: "Evidence from carbon -13 NMR for protonation of carbamyl-P and N-(phosphonacetyl)-L-aspartate in the active site of aspartate transcarbamylase"; & J. BIOL. CHEM., 1976, 251 (19), 5976-85 ----- | 1 | TECHNICAL FIELDS SEARCHED (Int. Cl.4)<br>C 07 F 9/38<br>C 07 F 9/40 |

The present search report has been drawn up for all claims

| Place of search | Date of completion of the search | Examiner |
|---|---|---|
| BERLIN | 05-10-1988 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)